(19) Europäisches Patentamt
European Patent Office
Office européen des brevets

(11) Veröffentlichungsnummer: **0 204 229**
**B1**

# (12) EUROPÄISCHE PATENTSCHRIFT

(45) Veröffentlichungstag der Patentschrift: **18.01.89**

(51) Int. Cl.⁴: **C 07 C 79/35**, C 07 C 76/02

(21) Anmeldenummer: **86107039.9**

(22) Anmeldetag: **23.05.86**

## (54) Verfahren zur Herstellung von 2,4-Dinitrophenol-alkoxy-alkylethern.

(30) Priorität: **04.06.85 DE 3519983**

(43) Veröffentlichungstag der Anmeldung: **10.12.86 Patentblatt 86/50**

(45) Bekanntmachung des Hinweises auf die Patenterteilung: **18.01.89 Patentblatt 89/3**

(84) Benannte Vertragsstaaten: **BE CH DE FR GB IT LI**

(56) Entgegenhaltungen:
**EP-A- 0 011 048**
**DE-A- 3 437 665**
**US-A- 2 988 571**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr., Heinrich-Bleicher-Strasse 40, D-6000 Frankfurt am Main 50 (DE)**
Erfinder: **Schophoff, Friedrich, Dr., Konstanzer Strasse 64, D-6000 Frankfurt am Main 61 (DE)**

**EP 0 204 229 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 2,4-Dinitrophenol-alkoxyalkylethern durch Umsetzung von 2,4-Dinitrochlorbenzol mit Alkoholen in Gegenwart von Lithiumoxid oder -hydroxid.

2,4-Dinitrophenol-alkoxyalkylether der allgemeinen Formel (I)

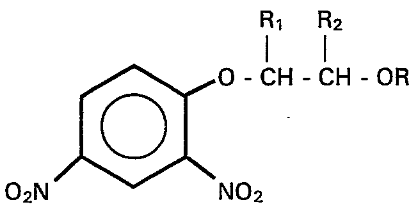

(I)

in welcher R einen ggf. durch $Alkoxy_{C_1-C_4}$gruppen substituierten $Alkyl_{C_1-C_4}$rest, einen Phenylrest welcher durch Alkyl- oder Alkoxygruppen von jeweils 1 bis 4 Kohlenstoffatomen oder durch Chlor- oder Bromatome substituiert sein kann, oder zusammen mit $R_2$ eine Alkylenbrücke $-(CH_2)_n-$, worin n die Zahl 3 oder 4 ist, bedeutet, $R_1$ ein Wasserstoffatom oder einen Alkylrest von 1 bis 4 Kohlenstoffatomen bedeutet, und $R_2$ ein Wasserstoffatom oder einen Alkylrest von 1 bis 4 Kohlenstoffatomen oder zusammen mit R eine Alkylenbrücke $-(CH_2)_n-$ darstellt, wobei n die genannte Bedeutung hat, sind technisch wichtige Vorprodukte zur Herstellung von Azo-Dispersionsfarbstoffen, wie sie beispielsweise in der Belgischen Patentschrift 634 032 beschrieben sind. Zu ihrer Herstellung sind zwei Verfahren vorgeschlagen worden:

1. Umsetzung von 2,4-Dinitrochlorbenzol mit einem Alkohol der Formel (II)

$R_1$ $R_2$
| |
HO-CH-CH-OR (II)

in welchen $R_1$, $R_2$ und R die vorstehend genannten Bedeutungen haben, in Gegenwart von Natriumhydroxid (EP-OS 11 048).

2. Umsetzung von 2,4-Dinitrochlorbenzol mit einem Alkalimetallalkoholat der Formel (III)

$R_1$ $R_2$
| |
Me-O-CH-CH-OR (III)

in welcher $R_1$, $R_2$ und R die vorstehend genannten Bedeutungen haben, und Me ein Alkalimetallatom darstellt (Deutsche Patentanmeldung P 3 437 665.8).

Die erstgenannte Umsetzung verläuft nur wenig selektiv und liefert, abhängig vom Molverhältnis 2,4-Dinitrochlorbenzol zum Alkohol der Formel (II) bis zu 10% unerwünschtes, weil schwierig abzutrennendes und kaum zu entsorgendes 2,4-Dinitrophenol. Bei Molverhältnissen 2,4-Dinitrochlorbenzol zum Alkohol der Formel (II) von 1 : ~5 kann zwar die 2,4-Dinitrophenol-Bildung auf Werte von 2 - 3% gesenkt werden, eine entscheidende Verbesserung ist damit aber nicht erreicht, weil sie auch in diesem Falle einen wünschenswerten Einsatz von ungereinigtem 2,4-Dinitrophenol-alkoxyalkylether (I) zu Folgereaktionen (katalytische Reduktion) nicht erlaubt und zudem wasserhaltige Regenerat-Alkohole der genannten Formel (II) liefert, die nur technisch äusserst aufwendig entwässert werden können, ohne diese Massnahme aber infolge ihres Wassergehaltes bei der Umsetzung mit 2,4-Dinitrochlorbenzol stark erhöhte 2,4-Dinitrophenol-Bildung zur Folge haben.

Die zweitgenannte Umsetzung beeinhaltet diese Probleme nicht, erfordert aber zur Herstellung des Alkalimetallalkoholats der genannten Formel (III) Alkalimetall als Ausgangsprodukt, für dessen technische Handhabung spezielle Apparaturen und eine aufwendige Abgas-Entsorgung (lösungsmittelhaltiger Wasserstoff) Vorbedingungen sind.

Es bestand somit ein Bedürfnis für ein technisch durchführbares Verfahren zur Herstellung von 2,4-Dinitrophenol-alkoxyalkylethern, das es gestattet, die Umsetzung ohne Verwendung von Alkalimetall so durchzuführen, dass keine die Folgereaktionen störenden Mengen an 2,4-Dinitrophenol gebildet werden, d.h., dass die unerwünschte Bildung von 2,4-Dinitrophenol auf unter 1,5% gesenkt wird. Dabei durfte ausserdem kein wasserhaltiger Regenerat-Alkohol der genannten Formel (II) anfallen, da dieser das genannte Ziel bei Recyclierung unerreichbar machen würde.

Es wurde nun überraschenderweise gefunden, dass man 2,4-Dinitrophenol-alkoxyalkylether der vorstehend genannten allgemeinen Formel (I), welche weniger als 1,5% unerwünschtes 2,4-Dinitrophenol enthalten, auf technisch einfache Weise herstellen kann, indem man 2,4-Dinitrochlorbenzol mit einem Alkohol der Formel (II)

$R_1$ $R_2$
| |
HO-CH-CH-OR (II)

in welcher $R_1$, $R_2$ und R die vorstehend genannten Bedeutungen haben, in Gegenwart von Lithiumoxid oder Lithiumhydroxid bei Temperaturen von 0°C bis 50°C, vorzugsweise 20°C bis 40°C, umsetzt, ggf. die Reaktionsmischung mit einer nicht flüchtigen, im Reaktionsmedium löslichen schwachen Säure neutralisiert und nachfolgend den im Überschuss vorhandenen Alkohol der Formel (II) abdestilliert, dann mit soviel Wasser von 20°C bis 80°C, vorzugsweise von 35°C bis 60°C, versetzt, dass das entstandene Lithiumchlorid vollständig in Lösung geht, und den sich in fester oder flüssiger Phase abscheidenden 2,4-Dinitrophenol-alkoxyalkylether von der wässrigen Salzphase abtrennt.

Das Lithiumhydroxid kann in wasserfreier Form oder in Form des kostengünstigeren Lithiumhydroxid-hydrats ($LiOH \cdot H_2O$) eingesetzt werden.

Bei Anwendung des wasserfreien Lithiumhydroxids gelingt es, bei Anwendung eines nur geringen Überschusses an Alkohol der genannten Formel (II) einen 2,4-Dinitrophenol-alkoxyalkylether der genannten Formel (I) herzustellen, der ohne weitere Reinigung für Folgereaktionen einsetzbar ist. Da

wegen der nahezu stöchiometrischen Umsetzung des 2,4-Dinitrochlorbenzols mit einem Alkohol der Formel (II) eine Regeneration des eingesetzten Alkohols entfällt, treten Probleme mit unerwünschtem wasserhaltigem Regenerat nicht auf, so dass nach dem erfindungsgemässen Verfahren die Zielprodukte der Formel (I) in technisch besonders einfacher Weise ohne aufwendige, spezielle Apparate und Manipulationen zugänglich werden.

Wendet man das Lithiumhydroxid-hydrat an, so muss der Alkohol der Formel (II) in mindestens 5-molarem Überschuss, bezogen auf eingesetztes 2,4-Dinitrochlorbenzol, eingesetzt werden. Bei der in diesem Fall erforderlichen destillativen Regeneration des überschüssigen Alkohols der Formel (II) resultiert ein wasserhaltiges Destillat, das von Zeit zu Zeit aufwendig vor dem Wiedereinsatz entwässert werden muss (Fraktionierung über vielbödige Kolonnen oder Schleppdestillation mit unpolaren Solventien, z.B. Cyclohexan oder Toluol), weshalb diese Verfahrensvariante weniger bevorzugt ist.

Das Verfahren wird im einzelnen in der Weise durchgeführt, dass man ein Gemisch aus 1 mol 2,4-Dinitrochlorbenzol und der 1,2- bis 10molaren, vorzugsweise 1,3- bis 1,7molaren Menge eines Alkohols der Formel (II) bei Temperaturen von 0°C bis 50°C, vorzugsweise von 20°C bis 40°C, innerhalb 0,5 bis 5 Stunden, vorzugsweise 1 bis 2 Stunden mit der 1- bis 1,2molaren, vorzugsweise 1,05- bis 1,1-molaren Menge Lithiumverbindung (1/2 $Li_2O$ oder LiOH bzw. $LiOH \cdot H_2O$) unter Rühren versetzt, 2 bis 10 Stunden, vorzugsweise 3 bis 5 Stunden nachrührt und ggf. nach Abdestillieren des überschüssigen Alkohols der Formel (II) aus der mit einer nicht flüchtigen, im Medium löslichen, schwachen Säure, wie beispielsweise Benzoesäure, Terephthalsäure, Isophthalsäure, neutralisierten Reaktionsmischung, mit soviel Wasser von 20°C bis 80°C, vorzugsweise von 35°C bis 60°C, versetzt, dass das gebildete Lithiumchlorid vollständig in Lösung geht, und den dabei in Form von Granalien oder als flüssige Phase sich abscheidenden 2,4-Dinitrophenol-alkoxyalkylether durch Filtration oder Phasentrennung von der wässrigen Salzphase abtrennt.

Man erhält hierbei in 98- bis 99%iger Ausbeute ein Produkt der genannten Formel (I), das praktisch frei von 2,4-Dinitrophenol ist und ohne Reinigung für Folgereaktionen (beispielsweise katalytische Reduktion) eingesetzt werden kann. Das gebildete Lithium-2,4-dinitrophenolat (maximal 1,5%, bezogen auf eingesetztes 2,4-Dinitrochlorbenzol) bleibt in der wässrigen Salzphase quantitativ gelöst, die ggf. nach Behandeln mit einem Adsorptionsmittel (Aktivkohle, synthetischem Adsorptionsharz vom XAD-Typ oder basischen mineralischen Adsorbentien), einer biologischen Kläranlage zugeführt werden kann.

Im Gegensatz dazu enthält eine nach dem Verfahren der weiter oben genannten EP-OS 11 048 hergestellte Verbindung der Formel (I) 3 bis 6% 2,4-Dinitrophenol in Form des Natriumsalzes, wodurch Folgereaktionen (beispielsweise katalytische Reduktion) ohne aufwendige Reinigung (z.B. mehrfaches Ausrühren mit der 20- bis 30fachen Menge Wasser) nicht durchführbar sind und die Reinigungslösungen infolge ihres hohen 2,4-Dinitrophenol-Gehaltes zusätzlich erhebliche Entsorgungsprobleme bereiten.

Das erfindungsgemässe Verfahren stellt somit gegenüber den bekannten Verfahren einen erheblichen technischen Fortschritt dar.

Die folgenden Beispiele sollen die Erfindung näher erläutern, ohne ihren Umfang darauf zu beschränken.

*Beispiel 1*

Zu einer gerührten Mischung aus 202,6 Teilen 2,4-Dinitrochlorbenzol und 116 Teilen Methylglykol gibt man innerhalb 90 Minuten portionsweise 25,2 Teile Lithiumhydroxid (wasserfrei) so zu, dass die Innentemperatur nicht über 35°C steigt. Der Reaktionsfortgang wird durch HPLC verfolgt (HPLC = high pressure liqid chromatography). Wenn kein 2,4-Dinitrochlorbenzol mehr nachweisbar ist (ca. 3 bis 5 Stunden), gibt man 750 Teile Wasser von 60°C zu, rührt 30 Minuten bei 40°C nach, kühlt auf 0°C bis 5°C ab und isoliert das in Granalien erstarrte 2,4-Dinitro-β-methoxyethoxybenzol durch Filtration, wäscht mit 250 Teilen Wasser und trocknet. Man erhält 237,2 Teile (98% der Theorie) Produkt vom Schmelzpunkt 52°C, in welchem 2,4-Dinitrophenol nicht mehr nachweisbar ist.

Im Filtrat (1100 Teile) lässt sich nach Neutralisation mit verdünnter Salzsäure durch HPLC ein Gehalt von 0,16% 2,4-Dinitrophenol feststellen, was einem 2,4-Dinitrophenol-Anfall von 1,8 Teilen pro Ansatz (0,98% der Theorie) entspricht.

*Beispiel 2*

Arbeitet man in der in Beispiel 1 angegebenen Weise, setzt aber anstelle der 25,2 Teile Lithiumhydroxid (wasserfrei) 15 Teile Lithiumoxid zu, so erhält man 239,5 Teile 2,4-Dinitro-β-methoxyethoxybenzol (99%% der Theorie) vom Schmelzpunkt 52,5°C, in welchem 2,4-Dinitrophenol nicht mehr nachweisbar ist.

Im Filtrat (1100 Teile) lassen sich nach Neutralisation mit Salzsäure durch HPLC 0,06% 2,4-Dinitrophenol feststellen (≙ 0,65 Teile pro Ansatz; 0,36% der Theorie).

*Beispiel 3*

Arbeitet man in der in Beispiel 1 angegebenen Weise, setzt aber anstelle der 25,2 Teile Lithiumhydroxid (wasserfrei) 45,2 Teile wasserhaltiges Lithiumhydroxid $LiOH \cdot H_2O$ ein, so erhält man 233,5 Teile 2,4-Dinitro-β-methoxyethoxybenzol (96,5% der Theorie) vom Schmelzpunkt 51,5°C, in welchem 2,4-Dinitrophenol gemäss HPLC zu 0,05% (0,12 Teile 2,4-Dinitrophenol; 0,06% der Theorie) noch enthalten ist.

Im Filtrat (1100 Teile) lassen sich nach Neutralisation mit Salzsäure durch HPLC 0,027% 2,4-Dinitrophenol nachweisen (≙ 3,00 Teile pro Ansatz; 1,63% der Theorie), so dass die gesamte 2,4-Dinitrophenol-Bildung 3,12 Teile pro Ansatz (≙ 1,69% der Theorie) beträgt.

*Beispiel 4*

Eine Mischung aus 202,6 Teilen 2,4-Dinitrochlorbenzol und 608,8 Teilen Methylglykol wird bei maximal 30°C innerhalb 30 Minuten mit 25,2 Teilen Lithiumhydroxid (wasserfrei) unter Rühren versetzt. Man rührt 3 Stunden bei 25°C nach, bis durch HPLC kein 2,4-Dinitrochlorbenzol mehr nachweisbar ist, gibt dann 6,7 Teile Benzoesäure zu und destilliert das überschüssige Methylglykol bei 100 mbar und einer maximalen Sumpftemperatur von 90°C weitgehend ab.

Der Rückstand wird nach Abkühlen auf 60°C mit 400 Teilen Wasser von 60°C verrührt. Anschliessend wird die organische Unterphase noch zweimal mit je 200 Teilen Wasser von 60°C gewaschen. Man erhält 237,8 Teile dinitrophenolfreie 2,4-Dinitro-β--methoxyethoxybenzol-Schmelze (98,3% der Theorie) mit einem Erstarrungspunkt von 50°C. In den vereinigten wässrigen Phasen (1112 Teile) lassen sich durch HPLC 0,08% 2,4-Dinitrophenol nachweisen (≙ 0,92 Teile pro Ansatz; 0,50% der Theorie).

*Beispiel 5*

Arbeitet man in der in Beispiel 4 angegebenen Weise, setzt aber anstelle der 25,2 Teile Lithiumhydroxid (wasserfrei) 45,2 Teile wasserhaltiges Lithiumhydroxid ($LiOH \cdot H_2O$) ein, so erhält man 236,0 Teile 2,4-Dinitro-β-methoxyethoxybenzol-Schmelze (97,5% der Theorie) vom Erstarrungspunkt 49,5°C, in welcher mittels HPLC kein 2,4-Dinitrophenol nachweisbar ist. In den vereinigten wässrigen Phasen (1130 Teile) lassen sich durch HPLC 0,15% 2,4-Dinitrophenol nachweisen (≙ 1,70 Teile pro Ansatz; 0,92% der Theorie).

*Beispiel 6*

Eine Mischung aus 202,6 Teilen 2,4-Dinitrochlorbenzol und 126 Teilen Ethylglykol wird unter Rühren bei maximal 30°C innerhalb 1 Stunde mit 25,0 Teilen Lithiumhydroxid (wasserfrei) portionsweise versetzt.

Man rührt nun so lange nach, bis durch HPLC im Reaktionsgemisch kein Dinitrochlorbenzol mehr nachweisbar ist (3-5 Stunden), gibt dann 750 Teile Wasser von 50°C zu, rührt 30 Minuten bei 50°C nach und trennt die organische Unterphase ab. Sie wird durch zweimaliges Ausrühren mit jeweils 250 Teilen Wasser von 50°C gewaschen. Man erhält nach Trocknen im Vakuum 251,0 Teile 2,4-Dinitro--β-ethoxyethoxybenzol (98,0% der Theorie), das bei Raumtemperatur flüssig ist und in dem durch HPLC kein 2,4-Dinitrophenol mehr nachweisbar ist.

In den vereinigten wässrigen Phasen (1200 Teile) lassen sich nach Neutralisation mit verdünnter Salzsäure durch HPLC 0,14% 2,4-Dinitrophenol nachweisen (≙ 1,82 Teile pro Ansatz; 0,99% der Theorie).

*Beispiele 7 bis 14*

Arbeitet man in der in Beispiel 6 angegebenen Weise, ersetzt aber das Ethylglykol durch aliquote Mengen der in der nachfolgenden Tabelle angegebenen Alkohole der genannten allgemeinen Formel (II), so erhält man in der aufgeführten Reaktionszeit die entsprechenden 2,4-Dinitrophenol-alkoxyalkylether der genannten allgemeinen Formel (I) in der in der Tabelle genannten Ausbeute. In keinem dieser erfindungsgemäss hergestellten isolierten Produkte lässt sich durch HPLC 2,4-Dinitrophenol nachweisen.

Die in den wässrigen Phasen durch HPLC analysierte Menge an 2,4-Dinitrophenol ist (in % der Theorie) ebenfalls in der Tabelle angegeben.

TABELLE

| Beispiel | Alkohol (II) | Reaktionszeit | Ausbeute | Schmelzpunkt | 2,4-Dinitrophenol-Bildung |
|---|---|---|---|---|---|
| 7 | n-Butylglykol | 5 | 97,9% | — | 1,10% |
| 8 | Methyldiglykol | 5 | 98,1% | 44-45°C | 1,1 % |
| 9 | Ethyldiglykol | 8 | 97,6% | — | 2,08% |
| 10 | n-Butyldiglykol | 10 | 97,1% | — | 1,21% |
| 11 | i-Propylglykol | 7 | 97,8% | — | 0,9 % |
| 12 | Phenylglykol | 8 | 98,0% | 61-62°C | 0,8 % |
| 13 | 3-Methoxybutanol | 8 | 97,2% | 42-43°C | 1,3 % |
| 14 | Tetrahydrofurfuryl-alkohol | 6 | 98,2% | 53-54°C | 1,1 % |

Die erhaltenen Reaktionsprodukte [der Formel (I) entsprechend] sind, wenn kein Schmelzpunkt angegeben ist, bei Raumtemperatur flüssig.

*Vergleichsbeispiel (gemäss EP-OS 11 048)*

Ersetzt man im erfindungsgemässen Beispiel 1 das Lithiumhydroxid (wasserfrei) durch 42 Teile Natriumhydroxid (wasserfrei in Form von Plätzchen und Prills) und arbeitet im übrigen in der angegebenen Weise, so erhält man 229,5 Teile granuliertes Reaktionsprodukt vom Schmelzpunkt 45°C bis 48°C, das nach HPLC 3% 2,4-Dinitrophenol enthält (6,9 Teile; 3,75% der Theorie). Die Ausbeute an 2,4-Dinitro-β-methoxyethoxybenzol beträgt demnach 222,6 Teile, entsprechend 92,0% der Theorie.

Im Filtrat (1100 Teile) lassen sich nach Ansäuern mit verdünnter Salzsäure durch HPLC 0,59% 2,4-Dinitrophenol feststellen. Dies entspricht einem Anfall von weiteren 6,5 Teilen (3,53% der Theorie) 2,4-Dinitrophenol, so dass die gesamte 2,4-Dinitrophenol-Bildung 13,4 Teile pro Ansatz (7,28% der Theorie) beträgt.

**Patentanspruch**

Verfahren zur Herstellung von 2,4-Dinitrophenolalkoxyalkylethern der allgemeinen Formel (I)

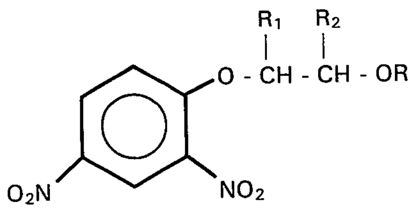

(I)

in welcher R einen ggf. durch Alkoxy$_{C_1\text{-}C_4}$gruppen substituierten Alkyl$_{C_1\text{-}C_4}$rest, einen Phenylrest, welcher durch Alkyl- oder Alkoxygruppen von jeweils 1 bis 4 Kohlenstoffatomen oder durch Chlor- oder Bromatome substituiert sein kann, oder zusammen mit $R_2$ eine Alkylenbrücke $-(CH_2)_n-$, worin n die Zahl 3 oder 4 ist, bedeutet, $R_1$ Wasserstoff oder einen Alkylrest von 1 bis 4 Kohlenstoffatomen bedeutet, und $R_2$ ein Wasserstoffatom oder einen Alkylrest von 1 bis 4 Kohlenstoffatomen oder zusammen mit R eine Alkylenbrücke $-(CH_2)_n-$ darstellt, wobei n die genannte Bedeutung hat, dadurch gekennzeichnet, dass man 2,4-Dinitrochlorbenzol mit einem Alkohol der Formel (II)

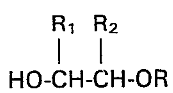

(II)

in welcher R, $R_1$ und $R_2$ die vorstehend genannten Bedeutungen haben, in Gegenwart von Lithiumoxid oder Lithiumhydroxid bei Temperaturen von 0°C bis 50°C umsetzt, ggf. die Reaktionsmischung mit einer nicht flüchtigen, im Reaktionsmedium löslichen, schwachen Säure neutralisiert, und nachfolgend den im Überschuss vorhandenen Alkohol der Formel (II) abdestilliert, dann mit soviel Wasser von 20°C bis 80°C versetzt, dass das entstandene Lithiumchlorid vollständig in Lösung geht, und den sich in fester oder flüssiger Phase abscheidenden 2,4-Dinitrophenol-alkoxyalkylether von der wässrigen Salzphase abtrennt.

**Claim**

A process for the preparation of 2,4-dinitrophenol alkoxyalkyl ethers of the formula (I)

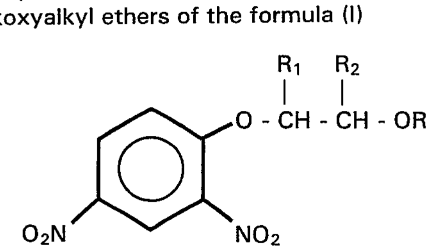

(I)

in which R denotes a $C_1$-$C_4$-alkyl radial which is optionally substituted by $C_1$-$C_4$-alkoxy groups, or a phenyl radical which can be substituted by alkyl or alkoxy groups having in each case 1 to 4 carbon atoms or by chlorine or bromine atoms, or, together with $R_2$, denotes an alkylene bridge $-(CH_2)_2-$ in which n is the number 3 or 4, $R_1$ denotes a hydrogen atom or an alkyl radical having 1 to 4 carbon atoms, and $R_2$ represents a hydrogenn atom or an alkyl radical having 1 to 4 carbon atoms, or, together with R, represents an alkylene bridge $-(CH_2)_n-$ in which n has the meaning mentioned, which comprises reacting 2,4-dinitrochlorobenzene with an alcohol of the formula (II)

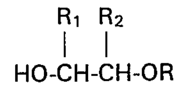

(II)

in which R, $R_1$ and $R_2$ have the meanings mentioned above, in the presence of lithium oxide or lithium hydroxide at temperatures from 0°C to 50°C, if appropriate neutralizing the reaction mixture with a non-volatile, weak acid which is soluble in the reaction medium, and subsequently removing by distillation the alcohol of the formula (II), present in excess, then adding sufficient water at 20°C to 80°C for the lithium chloride formed to dissolve completely, and separating off, from the aqueous salt phase, the 2,4-dinitrophenol alkoxyalkyl ether which has separated out in a solid or liquid phase.

**Revendication**

Procédé pour préparer des éthers alxoxyalkyliques du dinitro-2,4 phénol qui répondent à la formule générale (I)

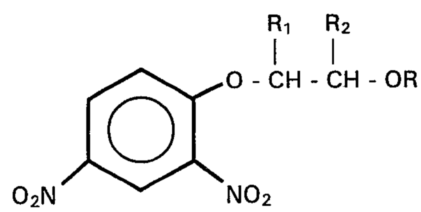

(I)

dans laquelle R représente un radical alkyle en $C_1$-$C_4$ éventuellement porteur de radicaux alcoxy en $C_1$-$C_4$, ou un radical phényle éventuellement porteur de radicaux alkyles ou alcoxy contenant chacun de 1 à 4 atomes de carbone ou d'atomes de chlore ou de brome, ou forme, avec $R_2$, un pont alkylène $-(CH_2)_n-$ dans lequel n désigne le nombre 3 ou le nombre 4, $R_1$ représente un atome d'hydrogène ou un radical alkyle contenant de 1 à 4 atomes de carbone, et $R_2$ représente un atome d'hydrogène ou un radical alkyle contenant de 1 à 4 atomes de carbone, ou forme, avec R, un pont alkylène $-(CH_2)_n-$ dans lequel n a la signification qui lui a été donnée ci-dessus, procédé caractérisé en ce qu'on fait réagir le dinitro-2,4 chloro-benzène avec un alcool répondant à la formule (II)

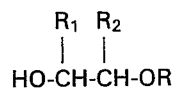

(II)

dans laquelle R, $R_1$ et $R_2$ ont les significations précédemment données, en présence d'oxyde de lithium ou d'hydroxyde de lithium, à des températures de

0°C à 50°C, éventuellement on neutralise le mélange réactionnel par un acide faible non volatil et soluble dans le milieu réactionnel, et ensuite on chasse par distillation l'alcool de formule (II) présent en excès, après quoi on ajoute une quantité d'eau à 20°C - 80°C suffisante pour que le chlorure de lithium formé passe totalement en solution, et on isole de la phase saline aqueuse l'éther alcoxy-alkylique du dinitro-2,4 phénol qui s'est séparé sous la forme d'une phase solide ou d'une phase liquide.